# EUROPEAN PATENT SPECIFICATION

(11) **EP 4 323 330 B1**
(45) Date of publication and mention of the grant of the patent: **10.12.2025**
(21) Application number: 22721968.0
(22) Date of filing: 15.04.2022
(51) Int. Cl.: C07C 231/14, C07C 231/24, C07C 235/60, C07D 265/26

(54) **PROCESS FOR THE PREPARATION OF HIGHLY PURE SALCAPROZIC ACID AND PHARMACEUTICALLY ACCEPTABLE SALTS THEREOF**
VERFAHREN ZUR HERSTELLUNG VON HOCHREINER SALCAPROZINSÄURE UND PHARMAZEUTISCH AKZEPTABLEN SALZEN DARAUS
PROCÉDÉ DE PRÉPARATION D'ACIDE SALCAPROZIQUE TRÈS PUR ET DE SES SELS PHARMACEUTIQUEMENT ACCEPTABLES

(30) Priority: 16.04.2021 US 202163176029 P; 13.04.2022 US 202217720007
(43) Date of publication of application: 21.02.2024
(73) Proprietor: Navinta III Inc, Boca Raton, FL 33487 (US)
(72) Inventor: PARMAR, Pankaj Vasudev, Vadodara, Gujarat 390023 (IN); MUTHIAH, Raja Jeyakumar John, Vadodara, Gujarat 390008 (IN); CHAUHAN, Vikas Kantilal, Vadodara, Gujarat 390023 (IN); SOLANKI, Jagdish Kanjibhai, Vadodara, Gujarat 390021 (IN)
(74) Representative: Barker Brettell LLP
(86) International application number: PCT/US2022/024963
(87) International publication number: WO 2022/221629

(56) References cited:
- WO-A1-00/59863
- WO-A1-2008/028859

## Description

### FIELD OF THE INVENTION

The present invention relates to a process for the preparation of Salcaprozic Acid and pharmaceutically acceptable salts thereof. The present invention also relates to a process for the purification of Salcaprozic Acid. More specifically, the process of the present invention comprises use of novel reagents for preventing the formation of colored impurity in Salcaprozic Acid and pharmaceutically acceptable salts thereof.

### BACKGROUND OF THE INVENTION

Many current commercial formulations containing peptides as active agents are delivered by a non-oral route, which is not convenient for patients. Use of N-[-8-(2-hydroxybenzoyl)amino] caprylic acid as an excipient which operates as a permeation enhancer or delivery agent is known to allow delivery of some peptides in an oral dosage form. It is believed that N-[-8-(2-hydroxybenzoyl)amino] caprylic acid temporarily alters the intestinal barrier to improve absorption of the peptide. N-[-8-(2-hydroxybenzoyl)amino] caprylic acid is also known as Salcaprozic Acid and is represented by formula A:

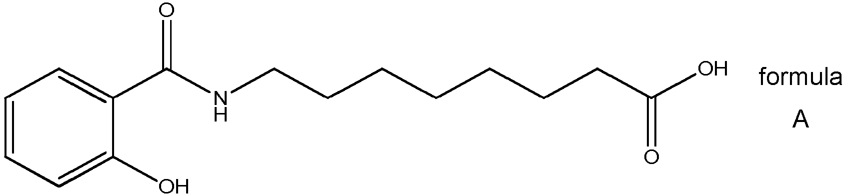

US Patent No. 5,650,386 discloses N-[-8-(2-hydroxybenzoyl)amino] caprylic acid or salts thereof, which can facilitate the delivery of various active agents with low oral bioavailability such as heparin, peptides, parathyroid hormone, bisphosphonates, vitamin B12.

US Patent No. 5,866,536 also discloses use of salts of N-[-8-(2-hydroxybenzoyl)amino] caprylic acid for delivering active agents such as heparin and calcitonin for oral administration.

U.S. Patent 9,278,123 (WO 2012/080471) discloses use of Salcaprozic Acid salts as a delivery agent in solid oral compositions of GLP-1 peptide. The PCT application discloses oral composition of Semaglutide with a monosodium salt of Salcaprozic Acid. In 2019, an oral tablet of Semaglutide in accordance with this patent and commercialized by Novo Nordisk was approved by the FDA.

General processes for the preparation of Salcaprozic Acid and its salts have also been described in the literature.

US Patent No. 5,650,386 teaches reaction of O-acetylsalicyl chloride with 8-aminooctanoic acid and 2-azacyclononanone respectively to yield Salcaprozic Acid.

A process according to WO 2000/59863 comprises coupling of 1,3-Benzoxazine 2,4-Dione (Carsalam) with ethyl-8-bromooctanoate followed by hydrolytic ring opening of the corresponding ethyl ester intermediate to yield Salcaprozic Acid.

WO 2001/092206 and WO 2008/028859 disclose processes for the preparation of N-[-8-(2-hydroxybenzoyl)amino] caprylic acid and a sodium salt thereof. Apart from the sodium salt, both applications disclose general base addition salts of N-[-8-(2-hydroxybenzoyl)amino] caprylic acid, such as alkali-metal salts (Na, K, Li), alkaline-earth metal salts (Mg, Ca, Ba), ammonium salts, basic amino acid salts (lysine, arginine), organic amines (dimethylamine, pyridine) and quaternary ammonium hydroxide salts (tetramethylammonium hydroxide).

In most of instances, the prior-art processes impart a pink color to the final Salcaprozic Acid or pharmaceutically acceptable salt thereof. WO 2008/028859 hypothesized that trace metals and/or oxygen are responsible for the formation of the pink color impurity, which is carried forward during salt formation and remains unaltered even after a number of purifications. The application discloses use of metal complexing agents like Ethylenediamine tetraacetic acid (EDTA), and reducing agents such as ascorbic acid (1%), Sodium bisulfite (NaHSO₃) (1%) and Triphenylphosphine (PPh₃) (0.1%) for removal of the unknown pink colored impurity from Salcaprozic Acid.

There is a desire to have alternative processes for preparing Salcaprozic Acid and its pharmaceutically acceptable salts that do not produce a pink color/impurity in the final product.

We have found that certain novel reagents can also be used for the removal of colored impurity/impurities from Salcaprozic Acid, and thus incorporation thereof in the process yields desired Salcaprozic Acid and pharmaceutically acceptable salts thereof without colored impurity.

### SUMMARY OF THE INVENTION

The present disclosure provides a processes for the preparation of Salcaprozic Acid and pharmaceutically acceptable salts thereof, which are free from colored impurity. The processes comprise use of novel reagents for removal of colored impurity.

In a first aspect, the present disclosure provides a process for the preparation of Salcaprozic Acid or a pharmaceutically acceptable salt thereof, comprising
hydrolyzing a compound of formula II: 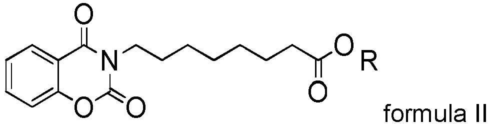
wherein, R is straight or branched chain alkyl
in the presence of benzotriazole, hydrazine, Sodium borohydride or mixture thereof; and isolating Salcaprozic Acid, which is free from colored impurity.

Preferably, the compound of formula II is 8-(2,4-dioxo-1,3-benzoxazin-3-yl)octanoic acid ethyl ester.

In certain embodiments, the compound of formula II is prepared from 1,3-Benzoxazine-2,4-dione. In certain of those embodiments, about 1% to about 5 % by weight of the benzotriazole, hydrazine, sodium borohydride or mixture thereof is used relative to weight of the 1,3-Benzoxazine-2,4-dione.

In some embodiments, the step of hydrolyzing occurs at ambient temperature.

In certain embodiments, the step of hydrolyzing comprises adding aqueous metal hydroxide and stirring at an elevated temperature up to about 95°C. In some of those embodiments, the process comprises cooling to 0-5°C followed by adjusting pH to about 9 to about 10. In certain of those embodiments, the process further comprises washing the pH adjusted solution with an organic solvent followed by discarding the organic washing solvent. In some of those embodiments, the step of isolating comprises lowering pH of the washed solution to obtain a wet solid Salcaprozic Acid.

In some embodiments, the process further comprises filtering and washing the isolated Salcaprozic Acid with a mixture of acetone and water.

In certain embodiments, the process further comprises contacting Salcaprozic Acid, which his free from colored impurity, with an organic or inorganic base in an organic solvent, water, or a mixture thereof to obtain a salt of Salcaprozic Acid. In some of those embodiments, absorbance at 400 nm of a 1% solution of the salt of Salcaprozic Acid is less than 0.03 and/or transmission at 600 nm of a 1% solution of the salt of Salcaprozic Acid is greater than 95%.

Also described is a process for removing a colored impurity from a Salcaprozic Acid salt comprising treating a solution of the salt with benzotriazole, hydrazine, sodium borohydride or a mixture thereof. Absorbance at 400 nm of a 1% solution of the treated salt is less than 0.03.

In one embodiment, the process for the preparation of Salcaprozic Acid or a pharmaceutically acceptable salt thereof, comprises
preparing 8-(2,4-dioxo-1,3-benzoxazin-3-yl)octanoic acid ethyl ester using 1,3-Benzoxazine-2,4-dione;
contacting the 8-(2,4-dioxo-1,3-benzoxazin-3-yl)octanoic acid ethyl ester with about 1% to about 5% by weight of benzotriazole, hydrazine, sodium borohydride or mixture thereof relative to the amount of 1,3-Benzoxazine-2,4-dione;
adding aqueous metal hydroxide and stirring at an elevated temperature up to about 95°C;
cooling to 0-5°C followed by adjusting pH to about 9 to about 10;
washing the pH adjusted solution with an organic solvent followed by discarding the organic washing solvent; and
lowering pH of the washed solution to isolate a wet solid Salcaprozic Acid.

In some embodiments, the step of contacting occurs at ambient temperature.

In certain embodiments, the process further comprises filtering and washing the wet solid Salcaprozic Acid with a mixture of acetone and water to obtain purified Salcaprozic Acid.

Advantageously, the processes disclosed herein produce Salcaprozic Acid that is free of or substantially free of colored impurity. That is, the Salcaprozic Acid has no detectable levels of trace metals and/or oxygen generated impurities that impart a pink color to the product. The Salcaprozic Acid produced according to this disclosure is considered to be colorless upon visual inspection or against a standard colorless solution. Salcaprozic Acid salts produced in accordance with this disclosure are white solids.

In certain preferred embodiments, an amount of any single impurity in the purified Salcaprozic Acid according to the processes described is less than 0.1%. Moreover, a 1% solution of a sodium salt of the Salcaprozic Acid made in accordance with this disclosure has absorbance at 400 nm of less than 0.02 or transmission at 600 nm of greater than 99%.

### DESCRIPTION OF THE DRAWINGS

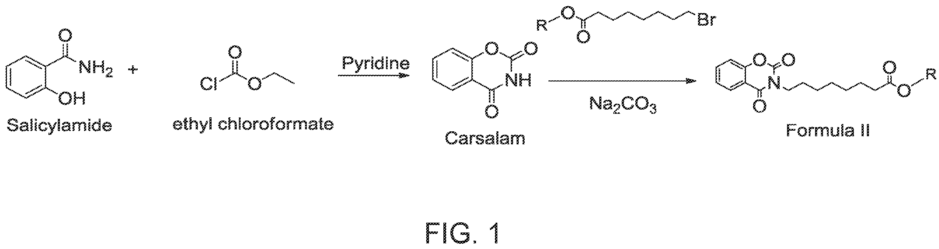

FIG. 1 shows a reaction scheme for preparation of a compound of formula II.

### DETAILED DESCRIPTION OF THE INVENTION

A wide range of reagents have been studied in order to achieve Salcaprozic Acid or a pharmaceutically acceptable salt thereof, which is essentially free from colored impurity. We have found three such novel reagents, which remove colored impurity and yield colorless Salcaprozic Acid or a pharmaceutically acceptable salt thereof as a white solid. Benzotriazole, hydrazine and sodium borohydride are such novel reagents of the present invention for the removal of colored impurity during the preparation of Salcaprozic Acid or pharmaceutically acceptable salts thereof.

The term "colored impurity" as used herein, refers to one impurity or more than one impurity or impurities that imparts a color to a substance, which may exist in amounts as low as a few parts per billion and still affect the color of substance. The "colored impurity" may be detected by visual inspection of a solid sample or its solution by reference or comparison to a standard colorless solid or solution.

The term "ambient temperature" means a temperature ranging from about 15°C to 40°C, preferably to a temperature ranging from about 20°C to 35°C.

The term "contacting" includes mixing, adding, slurring, stirring or a combination thereof. The term "contacting", as used herein for the purpose of reaction, is wherein one or more reagent(s) are mixed or added with each other, in the presence or absence of solvent(s) or an aqueous medium, in any sequence as a slurry or an insoluble solid mixture or stirred as a clear solution.

The term "about" is to be construed as modifying a term or value such that it is not an absolute. Such terms will be defined by the circumstances. This includes, at the very least, the degree of expected experimental error, technique error and instrument error for a given technique used to measure a value.

The term "comprising" and "comprises" mean the elements or steps as recited, or their equivalents in structure or function, plus any other element or elements which are not recited.

The terms "having" and "including" are also to be construed as open ended.

All ranges recited herein include the endpoints, including those that recite a range between two values. Whether so indicated or not, all values recited herein are approximate as defined by the circumstances, including the degree of expected experimental variation, technique variation, and instrument variation for a given technique used to measure a value.

The term "pharmaceutically acceptable salt" is to be construed as conventional base-addition salts that retain or improve the effectiveness or properties of Salcaprozic Acid and are formed from suitable non-toxic bases. The carboxylic acid (-COOH) and phenolic hydroxyl (-OH) moieties of Salcaprozic Acid may take part in converting to base addition salt. Sample base addition salts include, but are not limited to, inorganic or organic salts, for example alkali-metal salts such as sodium, potassium and lithium; alkaline-earth metal salts such as magnesium, calcium or barium; ammonium salts; basic amino acids, such as lysine or arginine; and organic amines, such as dimethylamine, triethylamine or pyridine. Based on the atom valances, the alkali-metal and alkaline-earth metal salts include, but not limited to, hemi-, mono-, di- and other multi-valent salts, such as, mono-sodium, di-sodium, mono-potassium, di-potassium, hemi-calcium, hemi-magnesium.

A process for the preparation of Salcaprozic Acid or a pharmaceutically acceptable salt thereof, comprises hydrolyzing a compound of formula II: 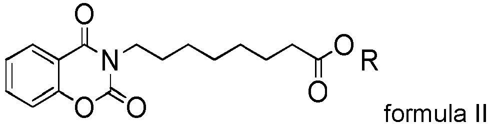 wherein, R is straight or branched chain alkyl in the presence of one or more novel reagents. The reagents for hydrolysis are selected from benzotriazole, hydrazine, and sodium borohydride or a mixture thereof.

The compound of formula II may be prepared according to methods known in art, or preferably by the process of FIG. 1. Reaction between Salicylamide and ethyl chloroformate is carried out in pyridine and methanol to obtain 1,3-Benzoxazine-2,4-dione (Carsalam). Coupling of 1,3-Benzoxazine-2,4-dione with an alkyl ester of 8-bromoctanoic acid is carried out in the presence of sodium carbonate as base and dimethyl acetamide as solvent to yield compound of formula **II.** Further, the compound of formula II may be isolated and optionally dried. Preferably, the compound of formula II is hydrolyzed without drying, as wet solid, to obtain Salcaprozic Acid.

In certain embodiments, the process includes contacting the hydrolyzed reaction mixture with one or more of the novel reagents.

The novel reagents are used in quantities sufficient to remove colored impurity from the Salcaprozic Acid, preferably from 0.1% to 10% w/w, more preferably from about 1% to about 5% by weight relative to amount of 1,3-Benzoxazine-2,4-dione.

Hydrolysis of the compound of formula II may be carried out in acidic or basic media; preferably, it is a base mediated hydrolysis, wherein the base is metal hydroxide selected from sodium, potassium, lithium or calcium hydroxides.

The hydrolysis may be carried out in an aqueous media at a temperature ranging from ambient temperature to reflux temperature and for the time sufficient to complete the hydrolysis of the compound of formula **II.**

In one preferred embodiment, the hydrolyzed reaction mixture comprises base addition salt of Salcaprozic Acid.

The hydrolyzed reaction mixture is acidified using aqueous hydrochloric acid to yield Salcaprozic Acid that is free from colored impurity. Alternatively, the hydrolyzed reaction mixture is first adjusted to an intermediate pH between 9 to 10 using aqueous hydrochloric acid followed by washing with an organic solvent selected from toluene, diisopropyl ether, dichloromethane or ethyl acetate. An obtained aqueous layer is further acidified using aqueous hydrochloric acid to yield Salcaprozic Acid that is free of colored impurity.

Before acidification of the hydrolyzed reaction mixture or an aqueous layer obtained after organic solvent washing, the hydrolyzed reaction mixture may be subjected to re-treatment with the novel reagent(s) to ensure complete removal of colored impurity.

A process of the present aspect may further comprise purification of Salcaprozic Acid in acetone:water to ensure complete removal of any other impurity.

In some embodiments, the purification includes suspending in acetone and stirring for about 5 hours at ambient temperature followed by addition of water at ambient temperature. The reaction mixture is filtered and washed with a mixture of acetone:water followed by drying to yield Salcaprozic Acid that is free from colored impurity as well as other process related impurities.

The process may further comprise conversion of colorless Salcaprozic Acid to its pharmaceutically acceptable salt(s). The pharmaceutically acceptable salts comprise but are not limited to sodium, potassium, calcium, magnesium, ammonium or organic amine addition salts. Preferably, Salcaprozic Acid is converted to corresponding sodium and potassium salts; more preferably, mono-sodium and mono-potassium.

A process for the conversion of Salcaprozic Acid to a pharmaceutically acceptable salt thereof comprises contacting Salcaprozic Acid with an organic or inorganic base in an organic solvent, water or a mixture thereof; and isolating a pharmaceutically acceptable salt of Salcaprozic Acid.

Alternatively, a process for the conversion of Salcaprozic Acid to a pharmaceutically acceptable salt thereof comprises contacting Salcaprozic Acid with an organic or inorganic base in an organic solvent, water or a mixture thereof to obtain a first salt of Salcaprozic Acid; and converting the first salt to a desired pharmaceutically acceptable salt of Salcaprozic Acid.

The selection of an organic or inorganic base is based on which pharmaceutically acceptable salt is desired. Preferably, an organic or an inorganic bases may be selected from sodium hydroxide, sodium methoxide, sodium ethoxide, potassium hydroxide, potassium methoxide, potassium ethoxide, calcium hydroxide, magnesium hydroxide or ammonium hydroxide; and organic bases may be selected from dimethylamine, triethylamine or pyridine. Preferably, the base is sodium hydroxide or potassium hydroxide and in the amount sufficient to obtain mono-sodium and mono-potassium salt, respectively.

The solvent is selected from methanol, ethanol or a mixture thereof. The pharmaceutically acceptable salt of Salcaprozic Acid is isolated by addition of a non-polar solvent, which acts as an anti-solvent. A non-polar solvent is preferably selected from heptane, diisopropylether, methyl tert-butyl ether, acetonitrile or a mixture thereof.

The processes of present invention utilizing benzotriazole, hydrazine and sodium borohydride yield better results as compared to prior art processes. If any of the contemplated reagents is not used in the process, it leads to Salcaprozic Acid with greater absorbance. Accordingly, the processes described herein are able to produce Salcaprozic Acid and salts thereof having absorbance at 400 nm of less than 0.03, preferably less than 0.02, most preferably about 0.01 or less, and a transmission at 600 nm of greater than 95%, more preferably greater than 98%, most preferably about 99% or greater, while maintaining high purity (e.g., greater than 99%).

### EXAMPLES

### Example 1: Preparation of Salcaprozic Acid using 2% Benzotriazole relative to 1,3-Benzoxazine 2,4-Dione

### Step 1: Preparation of 8-(2,4-dioxo-1,3-benzoxazin-3-yl)octanoic acid ethyl ester (Formula II)

1,3-Benzoxazine-2,4-dione (200 g) was added to dimethylacetamide (900mL) followed by addition of ethyl 8-bromo octanoate (292.5 g) in dimethylacetamide (100mL). The reaction mixture was allowed to stir for 30 minutes at 45-50°C and sodium carbonate (147.5 g) was added. The temperature was raised to 75-80°C and maintained until the reaction was complete. The reaction mixture was cooled to ambient temperature and water (1200mL) was charged. The solid was filtered and washed with water (400mL). The wet solid was subjected to slurry wash with water (1000mL) for 1 hour followed by filtration and washing with water (400mL) to obtain 612.5 g of wet 8-(2,4-dioxo-1,3-benzoxazin-3-yl)octanoic acid ethyl ester.

### Step 2: Hydrolysis of 8-(2,4-dioxo-1,3-benzoxazin-3-yl)octanoic acid ethyl ester (2% benzotriazole relative to 1,3-Benzoxazine 2,4-Dione):

Method A: Water (425mL) was charged to 8-(2,4-dioxo-1,3-benzoxazin-3-yl)octanoic acid ethyl ester prepared by the same process as in Step 1 (520.66 g wet solid was obtained from 170 gm 1,3-Benzoxazine 2,4-Dione) at ambient temperature followed by addition of benzotriazole (3.4 g) and stirred for 30 minutes. The reaction mixture was charged with aqueous sodium hydroxide solution (178.5 g NaOH in 714mL water) and stirred at 95°C for 120 minutes. The reaction mixture was cooled to 0-5°C followed by pH adjustment between 9 and 10 using concentrated HCl (153mL). The reaction mixture was brought to ambient temperature and aqueous layer was washed using toluene (850mL x 3), wherein toluene layer was discarded. The aqueous layer (1966 g) was further divided in two equal parts (983 g each) followed by further treatments according to Method A-1 or Method A-2.

### Step 3: Isolation of Salcaprozic Acid

Method A-1: One part of the aqueous layer (983 g) as obtained above was charged with sodium borohydride (1.7 g) and stirred for an hour at ambient temperature. The reaction mixture was added to pre-cooled aqueous HCl solution (179mL conc. HCl in 510mL water) and stirred for 30 minutes at 0-5°C. The solid was filtered, washed with water (170mL) and slurry washed with water (425mL) followed by filtration and washing with water (595mL). The wet Salcaprozic Acid solid, free from colored impurity was further purified as described in Step 4 below.

Method A-2: Second part of the aqueous layer (983 g) as obtained above was added to pre-cooled aqueous HCl solution (179mL conc. HCl in 510mL water) and stirred for 30 minutes at 0-5°C. The solid was filtered, washed with water (170mL) and slurry washed with water (425mL) followed by filtration and washing with water (595mL). The wet Salcaprozic Acid, free from colored impurity was further purified as described in Step 4 below.

### Step 4: Purification of Salcaprozic Acid

The wet Salcaprozic Acid obtained from Method A-1 was charged with acetone (425mL) and stirred for about 5 hours at ambient temperature. The reaction mixture was then diluted with water (850mL) and further stirred for about an hour at ambient temperature. The solid was filtered, washed with a mixture of acetone:water (85mL:170mL) and dried for 2 hours. The wet cake was unloaded and dried under vacuum at 50-55°C for 15 hours to obtain pure Salcaprozic Acid (109 g).

The wet Salcaprozic Acid obtained from Method A-2 was charged with acetone (425mL) and stirred for about 5 hours at ambient temperature. The reaction mixture was then charged water (850mL) and further stirred for about an hour at ambient temperature. The solid was filtered, washed with a mixture of acetone:water (85mL:170mL) and suck-dried for 2 hours. The wet cake was unloaded and dried under vacuum at 50-55°C for 15 hours to obtain pure Salcaprozic Acid (107 g).

### Example 2: Preparation of Salcaprozic Acid

### Step 1: Preparation of 8-(2,4-dioxo-1,3-benzoxazin-3-yl)octanoic acid ethyl ester (Formula II)

1,3-Benzoxazine-2,4-dione (500 g) was added to dimethylacetamide (2250mL) followed by addition of ethyl 8-bromo octanoate (731 g) in dimethylacetamide (250mL). The reaction mixture was allowed to stir for 30 minutes at 45-50°C and was added sodium carbonate (367.5 g). The temperature was raised to 75-80°C and maintained until the reaction was completed. The reaction mixture was cooled to ambient temperature and water (3000mL) was charged. The solid was filtered and washed with water (1000mL). The wet solid was subjected to slurry wash with water (2500mL) for 1 hour followed by filtration and washing with water (1000mL). The mixture was filtered to obtain 1486 g of wet solid of 8-(2,4-dioxo-1,3-benzoxazin-3-yl)octanoic acid ethyl ester (Formula II).

### Step 2: Hydrolysis

Wet solids of 8-(2,4-dioxo-1,3-benzoxazin-3-yl)octanoic acid ethyl ester obtained by the process of Step 1 were further hydrolyzed to Salcaprozic Acid by Method B, Method C, Method D, Method E, Method F & Method G as described below.

Method B: (5% benzotriazole relative to 1,3-Benzoxazine 2,4-Dione):

Water (50mL) was charged to 8-(2,4-dioxo-1,3-benzoxazin-3-yl)octanoic acid ethyl ester (29.72 g wet solid was obtained from 10 gm of 1,3-Benzoxazine-2,4-dione) at ambient temperature followed by addition of benzotriazole (0.5 g) and stirred for 30 minutes. The aqueous sodium hydroxide solution (10.5 g NaOH in 42mL water) was charged into reaction mass and stirred at 95°C for 120 minutes. The reaction mixture was cooled to 0-5°C followed by pH adjustment between 9 and 10 using concentrated HCl (7.5mL). The reaction mixture was brought to ambient temperature and aqueous layer was washed using toluene (50mL x 3), wherein toluene layer was discarded. The reaction mixture was charged into pre-cooled mixture of HCl (22.5mL conc. HCl in 60mL water) and stirred for 30 minutes at 0-5°C. The solid was filtered, washed with water (20mL). The wet solid was subjected to slurry wash with water (50mL) for 1 hour followed by filtration and washing with water (70mL) followed by suck-drying. The wet Salcaprozic Acid, free from colored impurity was further purified as described in Step 3 below..

### Method C: (1% benzotriazole relative to 1,3-Benzoxazine 2,4-Dione )

Water (125mL) was charged to 8-(2,4-dioxo-1,3-benzoxazin-3-yl)octanoic acid ethyl ester (74.3 g wet solid was obtained from 25 g of 1,3-Benzoxazine-2,4-dione) at ambient temperature followed by addition of benzotriazole (0.25g) and stirred for 30 minutes. The aqueous sodium hydroxide solution (26.25 g NaOH in 105mL water) charged into reaction mass and stirred at 95°C for 120 minutes. The reaction mixture was cooled to 0-5°C followed by pH adjustment between 9 and 10 using concentrated HCl (19mL). The reaction mixture was brought to ambient temperature and aqueous layer was washed using toluene (125mL x 3), wherein toluene layer was discarded.

The reaction mixture was charged into pre-cooled mixture of HCl (56mL conc. HCl in 150mL water) and stirred for 30 minutes at 0-5°C. The solid was filtered, washed with water (50mL). The wet solid was subjected to slurry wash with water (125mL) for 1 hour followed by filtration and washing with water (175mL) followed by suck-drying. The wet solid Salcaprozic Acid, free from colored impurity was further purified as described in Step 3 below

### Method D: (1% hydrazine relative to 1,3-Benzoxazine 2,4-Dione)

Water (50mL) was charged to 8-(2,4-dioxo-1,3-benzoxazin-3-yl)octanoic acid ethyl ester (29.72 g wet solid was obtained from 10 g of 1,3-Benzoxazine-2,4-dione) at ambient temperature followed by addition of hydrazine (0.1 g) and stirred for 30 minutes. An aqueous sodium hydroxide solution (10.5 g NaOH in 42mL water) was charged into reaction mass and stirred at 95°C for 120 minutes. The reaction mixture was cooled to 0-5°C followed by pH adjustment between 9 and 10 using concentrated HCl (7.5mL). The reaction mixture was brought to ambient temperature and aqueous layer was washed using toluene (50mL x 3), wherein toluene layer was discarded. The reaction mixture was charged into pre-cooled mixture of HCl (22.5mL conc. HCl in 60mL water) and stirred for 30 minutes at 0-5°C. The solid was filtered, washed with water (20mL). The wet solid was subjected to slurry wash with water (50mL) for 1 hour followed by filtration and washing with water (70mL) followed by suck-drying. The wet Salcaprozic Acid solid, free from colored impurity was further purified as described in Step 3 below

### Method E: (2% hydrazine relative to 1,3-Benzoxazine 2,4-Dione)

Water (50mL) was charged to 8-(2,4-dioxo-1,3-benzoxazin-3-yl)octanoic acid ethyl ester (29.72 g wet solid was obtained from 10 g of 1,3-Benzoxazine-2,4-dione) at ambient temperature followed by addition of hydrazine (0.2 g) and stirred for 30 minutes. The aqueous sodium hydroxide solution (178.5 g NaOH in 714mL water) charged into reaction mass and stirred at 95°C for 120 minutes. The reaction mixture was cooled to 0-5°C followed by pH adjustment between 9 and 10 using concentrated HCl (7.5mL). The reaction mixture was brought to ambient temperature and aqueous layer was washed using toluene (50mL x 3), wherein toluene layer was discarded. The reaction mixture was charged into pre-cooled mixture of HCl (22.5mL conc. HCl in 60mL water) and stirred for 30 minutes at 0-5°C. The solid was filtered, washed with water (20mL). The wet solid was subjected to slurry wash with water (50mL) for 1 hour followed by filtration and washing with water (70mL) followed by suck-drying. The wet Salcaprozic Acid solid , free from colored impurity was further purified as described in Step 3 below

### Method F: (5% hydrazine relative to 1,3-Benzoxazine 2,4-Dione)

Water (50mL) was charged to 8-(2,4-dioxo-1,3-benzoxazin-3-yl)octanoic acid ethyl ester (29.72 g wet solid was obtained from 10g of 1,3-Benzoxazine-2,4-dione) at ambient temperature followed by addition of hydrazine (0.5 g) and stirred for 30 minutes. The reaction mixture was charged with aqueous sodium hydroxide solution (10.5 g NaOH in 42mL water) and stirred at 95°C for 120 minutes. The reaction mixture was cooled to 0-5°C followed by pH adjustment between 9 and 10 using concentrated HCl (7.5mL). The reaction mixture was brought to ambient temperature and aqueous layer was washed using toluene (50mL x 3), wherein toluene layer was discarded. The reaction mixture was charged to pre-cooled mixture of HCl (22.5mL conc. HCl in 60mL water) and stirred for 30 minutes at 0-5°C. The solid was filtered, washed with water (20mL). The wet solid was subjected to slurry wash with water (50mL) for 1 hour followed by filtration and washing with water (70mL) followed by suck-drying. The wet Salcaprozic Acid solid, free from colored impurity was further purified as described in Step 3 below

### Method G: (total 4% Sodium Borohydride relative to 1,3-Benzoxazine 2,4-Dione)

Water (50mL) was charged to 8-(2,4-dioxo-1,3-benzoxazin-3-yl)octanoic acid ethyl ester (104 g wet solid was obtained from 35 g of 1,3-Benzoxazine-2,4-dione) at ambient temperature followed by addition of sodium borohydride (0.7 g) and stirred for 30 minutes. The aqueous sodium hydroxide solution (36.75 g NaOH in 147mL water) was charged into reaction mass and stirred at 95°C for 120 minutes. The reaction mixture was cooled to 0-5°C followed by pH adjustment between 9 and 10 using concentrated HCl (26mL). The reaction mixture was brought to ambient temperature and aqueous layer was washed using toluene (175mL x 3), wherein toluene layer was discarded. Charge sodium borohydride (0.7 g) into the reaction mixture and stir for 60 min at ambient temperature. The reaction mixture was charged into pre-cooled mixture of HCl (79mL conc. HCl in 210mL water) and stirred for 30 minutes at 0-5°C. The solid was filtered, washed with water (70mL). The wet solid was subjected to slurry wash with water (175mL) for 1 hour followed by filtration and washing with water (245mL) followed by suck-drying. The wet Salcaprozic Acid solid, free from colored impurity was further purified as described in Step 3 below.

### Step 3: Purification of Salcaprozic Acid

The wet Salcaprozic Acid obtained above in Method B was charged with acetone (50mL) and stirred for about 5 hours at ambient temperature. The reaction mixture was then charged with water (100mL) and further stirred for about an hour at ambient temperature. The solid was filtered, washed with a mixture of acetone:water (10mL:20mL) and suck-dried for 2 hours. The wet cake was unloaded and dried under vacuum at 50-55°C for 12 hours to obtain pure Salcaprozic Acid (12.9 g).

The wet Salcaprozic Acid obtained above in Method C was charged acetone (125mL) and stirred for about 5 hours at ambient temperature. The reaction mixture was then charged with water (250mL) and further stirred for about an hour at ambient temperature. The solid was filtered, washed with a mixture of acetone:water (25mL:50mL) and suck-dried for 2 hours. The wet cake was unloaded and dried under vacuum at 50-55°C for 12 hours to obtain pure Salcaprozic Acid (28.5 g).

The wet Salcaprozic Acid obtained above in Method D was charged in acetone (50mL) and stirred for about 5 hours at ambient temperature. The reaction mixture was then charged with water (100mL) and further stirred for about an hour at ambient temperature. The solid was filtered, washed with a mixture of acetone:water (10mL:20mL) and suck-dried for 2 hours. The wet cake was unloaded and dried under vacuum at 50-55°C for 12 hours to obtain pure Salcaprozic Acid (13.5 g).

The wet Salcaprozic Acid obtained above in Method E was charged acetone (50mL) and stirred for about 5 hours at ambient temperature. The reaction mixture was then charged water (100mL) and further stirred for about an hour at ambient temperature. The solid was filtered, washed with a mixture of acetone:water (10mL:20mL) and suck-dried for 2 hours. The wet cake was unloaded and dried under vacuum at 50-55°C for 12 hours to obtain pure Salcaprozic Acid (13.0 g).

The wet Salcaprozic Acid obtained above in Method F was charged acetone (50mL) and stirred for about 5 hours at ambient temperature. The reaction mixture was then charged water (100mL) and further stirred for about an hour at ambient temperature. The solid was filtered, washed with a mixture of acetone:water (10mL:20mL) and suck-dried for 2 hours. The wet cake was unloaded and dried under vacuum at 50-55°C for 12 hours to obtain pure Salcaprozic Acid (12.7 g).

The wet Salcaprozic Acid obtained above in Method G was charged acetone (175mL) and stirred for about 5 hours at ambient temperature. The reaction mixture was then charged water (350mL) and further stirred for about an hour at ambient temperature. The solid was filtered, washed with a mixture of acetone:water (35mL:70mL) and suck-dried for 2 hours. The wet cake was unloaded and dried under vacuum at 50-55°C for 12 hours to obtain pure Salcaprozic Acid (47.03 g).

### Example 3: Preparation of sodium salt of Salcaprozic Acid (SNAC)

An aqueous sodium hydroxide solution (15.04 g NaOH in 35mL water) was slowly added to a solution of Purified Salcaprozic Acid (100 g) (Purified Salcaprozic Acid obtained as in Example-1: Method-A2, Step-4) in ethanol (300mL), and stirred for 20 minutes below 35°C. Reaction mixture was diluted by addition of ethanol (300mL) and further stirred for 30 minutes. Heptane (360mL) was added as anti-solvent and stirred for 30 minutes at 10-15°C. The resulting reaction mixture was filtered and solid was washed with heptane (2 x 100mL). The resulting sodium salt of Salcaprozic Acid (87 g) (SNAC) was dried under vacuum at 60-65°C for 12-15 hours. HPLC Purity: 99.92 %, Absorbance (at 400nm): 0.01 and % of Transmitance : 99.42.

### Example 4: Comparative Preparation of Salcaprozic Acid without using additional reagents for removing colored impurity

Water (50mL) was charged to 8-(2,4-dioxo-1,3-benzoxazin-3-yl)octanoic acid ethyl ester (118.88 g wet solid was obtained from 40 g of 1,3-Benzoxazine-2,4-dione using the process of Example 1, Step 1) at ambient temperature and stirred for 30 minutes. The reaction mixture was charged into sodium hydroxide solution (42 g NaOH in 168mL water) and stirred at 95°C for 120 minutes. The reaction mixture was cooled to 0-5°C followed by pH adjustment between 9 and 10 using concentrated HCl (30mL). The reaction mixture was brought to ambient temperature and aqueous layer was washed using toluene (200mL x 3), wherein toluene layer was discarded. The reaction mixture was charged into pre-cooled mixture of HCl (90mL conc. HCl in 240mL water) and stirred for 30 minutes at 0-5°C. The solid was filtered, washed with water (80mL). The wet solid was subjected to slurry wash with water (200mL) for 1 hour followed by filtration and washing with water (280mL) followed by suck-drying. Weight of the wet cake of colored Salcaprozic Acid was 96 g.

### Example 5: Purification/ Reprocess of colored Salcaprozic Acid (obtained from example 4) using 2% Sodium borohydride/Hydrazine/Benzotriazole of 1,3-Benzoxazine 2,4-Dione

Wet Salcaprozic Acid (24 g wet solid made by process of Example 4 from 10 g of 1,3-Benzoxazine-2,4-dione) was charged into Sodium hydroxide solution (5 g Sodium hydroxide in 50mL water) and stirred for 10 to 15 minutes at ambient temperature. The reaction mixture was treated with hydrazine, sodium borohydride, or benzotriazole (0.2 g) and further stirred for about 60 to 70 minutes at ambient temperature. The reaction mass was cooled to 10°C to 15°C. The solid was isolated by addition of dilute hydrochloric acid solution. The solid was filtered, washed water (10mL). The wet solid was subjected to slurry wash with water (50mL) for 1 hour followed by filtration and washing with water (20mL) followed by suck-drying. The wet cake was unloaded and dried under vacuum at 50-55°C for 12 hours to obtain pure colorless Salcaprozic Acid.

### Example 6: Preparation of potassium salt of Salcaprozic Acid

Salcaprozic Acid (100 g) was charged to a solution of potassium hydroxide (1.1 eq; 22.09 g) in ethanol (266mL) at 60-65°C. The reaction temperature was maintained for 1 hour followed by hot filtration through 0.2 micron. The reaction mixture was cooled to 25-30°C. Diisopropylether (2857mL) was added to the reaction mixture followed by stirring for 60-90 minutes at 25-35°C. The resulting reaction mixture was filtered and potassium salt of Salcaprozic Acid was washed with diisopropylether (2 x 200mL). The solid was dried under vacuum at 60-65°C for 24 hours to obtain potassium salt of Salcaprozic Acid (90 g).

### Example 7: Comparative Data

Absorbance and Transmittance of 1% solution of sodium salt of Salcaprozic Acid was checked at 400nm and 600 nm respectively using UV-Vis Spectrophotometer (Perkin Elmer Lambda 365), The absorbance, transmittance and purity of the sodium salt of Salcaprozic Acid prepared according to various methods is shown in Table 1.

**TABLE 1**

| **Color and Clarity Data for Sodium Salt of Salcaprozic Acid** | | | | **HPLC Purity data of Salcaprozic Acid** | |
|---|---|---|---|---|---|
| **Process/ Reference** | **Experiment details** | **Absorbance (at 400nm)** | **% of Transmission** | **Purity (%)** | **Single maximum impurity** (%) |
| Prior art process from EP2064175B1 | Prior art process using EDTA | 0.023 | 98.39 | 99.73 | 0.17 |
| Original Process without any treatment | Example-4: Experiment performed without any treatment | 0.38 | 94.82 | - | - |
| Developed process of present invention after Purification Step | Method A-1: using benzotriazole (2%) and Sodium borohydride (2%) | 0.01 | 99.34 | 99.88 | 0.04 |
| | Method A-2: using benzotriazole (2%) | 0.01 | 99.42 | 99.88 | 0.05 |
| | Method-B: using benzotriazole (5%) | 0.01 | 99.19 | 99.89 | 0.07 |
| | Method-C: using benzotriazole (1%) | 0.01 | 99.30 | 99.89 | 0.06 |
| | Method-D: using hydrazine (1%) | 0.01 | 99.31 | 99.94 | 0.02 |
| | Method-E: using hydrazine (2%) | 0.01 | 99.38 | 99.93 | 0.03 |
| | Method-F: using hydrazine (5%) | 0.01 | 99.63 | 99.92 | 0.03 |
| | Method-G: using sodium borohydride (4%) | 0.01 | 99.30 | 99.92 | 0.05 |

From above data, it is evident that the process of present invention of using novel reagents benzotriazole, hydrazine and sodium borohydride gives better results as compared to prior art process. Also, as per example 4, if any reagent is not used in the process, it leads to Salcaprozic Acid with greater absorbance.

## Claims

1. A process for the preparation of Salcaprozic Acid or a pharmaceutically acceptable salt thereof, comprising:
a. hydrolyzing a compound of formula II: 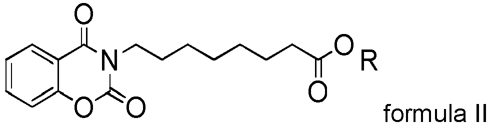 wherein, R is straight or branched chain alkyl
in the presence of benzotriazole, hydrazine, sodium borohydride or mixture thereof; and
b. isolating Salcaprozic Acid, which is free from colored impurity.

2. The process of claim 1, wherein the compound of formula II is prepared from 1,3-Benzoxazine-2,4-dione and the compound of formula II is hydrolyzed in presence of:
a) about 0.1% to about 10% by weight of the benzotriazole, hydrazine, sodium borohydride or mixture thereof relative to weight of the 1,3-Benzoxazine-2,4-dione; or
b) about 1% to about 5% by weight of the benzotriazole, hydrazine, sodium borohydride or mixture thereof is used relative to weight of the 1,3-Benzoxazine-2,4-dione.

3. The process of any of the preceding claims, wherein the step of hydrolyzing in the presence of benzotriazole, hydrazine, sodium borohydride or mixture thereof occurs at a temperature ranging from about 15°C to 40°C.

4. The process of any of the preceding claims, wherein the step of hydrolyzing comprises adding aqueous metal hydroxide and stirring at an elevated temperature up to about 95°C.

5. The process of any of the preceding claims, further comprising cooling to 0-5°C followed by adjusting pH to about 9 to about 10, and washing the pH adjusted solution with an organic solvent followed by discarding the organic washing solvent.

6. The process of claim 5, wherein the step of isolating comprises lowering pH of the washed solution to obtain a wet solid Salcaprozic Acid.

7. The process of any of the preceding claims, further comprising filtering and washing the isolated Salcaprozic Acid with a mixture of acetone and water.

8. The process of any of the preceding claims, further comprising contacting Salcaprozic Acid, which is free from colored impurity, with an organic or inorganic base in an organic solvent, water, or a mixture thereof to obtain a salt of Salcaprozic Acid.

9. The process of claim 8, wherein:
a) absorbance at 400 nm of a 1% solution of the salt of Salcaprozic Acid is less than 0.03, such as less than 0.02, preferably about 0.01 or less; and/or
b) transmission at 600 nm of a 1% solution of the salt of Salcaprozic Acid is greater than 95%, such as greater than 98%, preferably about 99% or greater.

10. A process for removing a colored impurity from a Salcaprozic Acid salt comprising treating a solution of the salt with benzotriazole, hydrazine, sodium borohydride or a mixture thereof.

11. The process of claim 10, wherein absorbance at 400 nm of a 1% solution of the treated salt is less than 0.03, such as less than 0.02, preferably about 0.01 or less.

12. The process of any preceding claim, comprising:
a. preparing 8-(2,4-dioxo-1,3-benzoxazin-3-yl)octanoic acid ethyl ester using 1,3-Benzoxazine-2,4-dione
b. contacting the 8-(2,4-dioxo-1,3-benzoxazin-3-yl)octanoic acid ethyl ester with about 1% to about 5% by weight of benzotriazole, hydrazine, Sodium borohydride or mixture thereof relative to the amount of 1,3-Benzoxazine-2,4-dione;
c. adding aqueous metal hydroxide and stirring at an elevated temperature up to about 95°C;
d. cooling to 0-5°C followed by adjusting pH to about 9 to about 10;
e. washing the pH adjusted solution with an organic solvent to remove byproducts and impurities; and
f. lowering pH of the washed solution to isolate a wet solid Salcaprozic Acid.

13. The process of claim 12, wherein the step of contacting occurs at a temperature ranging from about 15°C to 40°C.

14. The process of claim 12 or 13, further comprising:
g. filtering and washing the wet solid Salcaprozic Acid with a mixture of acetone and water to obtain purified Salcaprozic Acid,
wherein optionally an amount of any single impurity in the purified Salcaprozic Acid is less than 0.1%.

15. The process of any of the claims 12-14, wherein:
a) the Salcaprozic Acid is free of colored impurity; and/or
b) 1% solution of a sodium salt of the wet Salcaprozic Acid has absorbance at 400 nm of less than 0.03, such as less than 0.02, preferably about 0.01 or less, or transmission at 600 nm of greater than 95%, such as greater than 98%, preferably about 99% or greater.

## Patentansprüche

1. Verfahren zur Herstellung von Salcaprozinsäure oder einem pharmazeutisch akzeptablen Salz davon, umfassend:
a. Hydrolysieren einer Verbindung der Formel II: 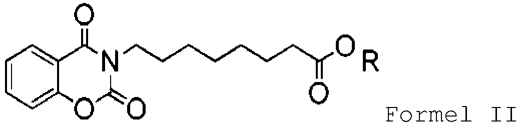 wobei R ein geradkettiges oder verzweigtes Alkyl ist, in Gegenwart von Benzotriazol, Hydrazin, Natriumborhydrid oder einer Mischung davon; und
b. Isolieren von Salcaprozinsäure, die frei von farbigen Verunreinigungen ist.

2. Verfahren nach Anspruch 1, wobei die Verbindung der Formel II aus 1,3-Benzoxazin-2,4-dion hergestellt wird und die Verbindung der Formel II in Gegenwart von:
a) etwa 0,1 bis etwa 10 Gew.-% Benzotriazol, Hydrazin, Natriumborhydrid oder einer Mischung davon, bezogen auf das Gewicht des 1,3-Benzoxazin-2,4-dions, hydrolysiert wird; oder
b) etwa 1 bis etwa 5 Gew.-% Benzotriazol, Hydrazin, Natriumborhydrid oder einer Mischung davon, bezogen auf das Gewicht des 1,3-Benzoxazin-2,4-dions, verwendet wird.

3. Verfahren nach einem der vorhergehenden Ansprüche, wobei der Schritt von Hydrolysieren in Gegenwart von Benzotriazol, Hydrazin, Natriumborhydrid oder einer Mischung davon bei einer Temperatur zwischen 15°C und 40°C erfolgt.

4. Verfahren nach einem der vorhergehenden Ansprüche, wobei der Schritt von Hydrolysieren Hinzufügen von wässrigem Metallhydroxid und Rühren bei einer erhöhten Temperatur von bis zu etwa 95°C umfasst.

5. Verfahren nach einem der vorhergehenden Ansprüche, ferner umfassend Abkühlen auf 0-5°C und anschließendes Einstellen des pH-Werts auf etwa 9 bis etwa 10 sowie Waschen der pH-eingestellten Lösung mit einem organischen Lösungsmittel und anschließendes Verwerfen des organischen Waschlösungsmittels.

6. Verfahren nach Anspruch 5, wobei der Schritt von Isolieren Senken des pH-Werts der gewaschenen Lösung umfasst, um eine nasse, feste Salcaprozinsäure zu erhalten.

7. Verfahren nach einem der vorhergehenden Ansprüche, ferner umfassend Filtern und Waschen der isolierten Salcaprozinsäure mit einer Mischung aus Aceton und Wasser.

8. Verfahren nach einem der vorhergehenden Ansprüche, ferner umfassend Inkontaktbringen von Salcaprozinsäure, die frei von farbigen Verunreinigungen ist, mit einer organischen oder anorganischen Base in einem organischen Lösungsmittel, Wasser oder einer Mischung davon, um ein Salz der Salcaprozinsäure zu erhalten.

9. Verfahren nach Anspruch 8, wobei:
a) die Absorption bei 400 nm einer 1 %igen Lösung des Salzes der Salcaprozinsäure weniger als 0,03, beispielsweise weniger als 0,02, vorzugsweise etwa 0,01 oder weniger beträgt; und/oder
b) die Transmission bei 600 nm einer 1 %igen Lösung des Salzes der Salcaprozinsäure größer als 95 %, beispielsweise größer als 98 %, vorzugsweise etwa 99 % oder größer ist.

10. Verfahren zum Entfernen einer farbigen Verunreinigung aus einem Salcaprozinsäuresalz, umfassend Behandeln einer Lösung des Salzes mit Benzotriazol, Hydrazin, Natriumborhydrid oder einer Mischung davon.

11. Verfahren nach Anspruch 10, wobei die Absorption bei 400 nm einer 1 %igen Lösung des behandelten Salzes weniger als 0,03, beispielsweise weniger als 0,02, vorzugsweise etwa 0,01 oder weniger beträgt.

12. Verfahren nach einem der vorhergehenden Ansprüche, umfassend:
a. Herstellen von 8-(2,4-Dioxo-1,3-benzoxazin-3-yl)octansäureethylester unter Verwendung von 1,3-Benzoxazin-2,4-dion
b. Inkontaktbringen des 8-(2,4-Dioxo-1,3-benzoxazin-3-yl)octansäureethylesters mit etwa 1 bis etwa 5 Gew.-% Benzotriazol, Hydrazin, Natriumborhydrid oder einer Mischung davon, bezogen auf die Menge an 1,3-Benzoxazin-2,4-dion;
c. Hinzufügen von wässrigem Metallhydroxid und Rühren bei einer erhöhten Temperatur von bis zu etwa 95°C;
d. Abkühlen auf 0-5°C, gefolgt von Einstellen des pH-Werts auf etwa 9 bis etwa 10;
e. Waschen der pH-eingestellten Lösung mit einem organischen Lösungsmittel, um Nebenprodukte und Verunreinigungen zu entfernen; und
f. Senken des pH-Werts der gewaschenen Lösung, um eine nasse, feste Salcaprozinsäure zu isolieren.

13. Verfahren nach Anspruch 12, wobei der Schritt von Inkontaktbringen bei einer Temperatur zwischen etwa 15°C und 40°C erfolgt.

14. Verfahren nach Anspruch 12 oder 13, ferner umfassend:
g. Filtern und Waschen der nassen, festen Salcaprozinsäure mit einer Mischung aus Aceton und Wasser, um gereinigte Salcaprozinsäure zu erhalten,
wobei optional eine Menge einer einzelnen Verunreinigung in der gereinigten Salcaprozinsäure weniger als 0,1 % beträgt.

15. Verfahren nach einem der Ansprüche 12-14, wobei:
a) die Salcaprozinsäure frei von farbigen Verunreinigungen ist; und/oder
b) eine 1 %ige Lösung eines Natriumsalzes der nassen Salcaprozinsäure eine Absorption bei 400 nm von weniger als 0,03, beispielsweise weniger als 0,02, vorzugsweise etwa 0,01 oder weniger, oder eine Transmission bei 600 nm von mehr als 95 %, beispielsweise mehr als 98 %, vorzugsweise etwa 99 % oder mehr, aufweist.

## Revendications

1. Procédé de préparation d'acide salcaprozique ou d'un sel pharmaceutiquement acceptable de celui-ci, comprenant :
a. l'hydrolyse d'un composé de formule II : 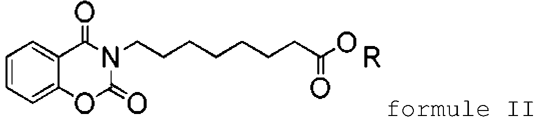 dans lequel R est un alkyle à chaîne droite ou ramifiée en présence de benzotriazole, d'hydrazine, de borohydrure de sodium ou d'un mélange de ceux-ci ; et
b. l'isolement de l'acide salcaprozique, qui est exempt d'impuretés colorées.

2. Procédé selon la revendication 1, dans lequel le composé de formule II est préparé à partir de 1,3-benzoxazine-2,4-dione et le composé de formule II est hydrolysé en présence de :
a) environ 0,1 % à environ 10 % en poids de benzotriazole, d'hydrazine, de borohydrure de sodium ou d'un mélange de ceux-ci par rapport au poids de 1,3-benzoxazine-2,4-dione ; ou
b) environ 1 % à environ 5 % en poids de benzotriazole, d'hydrazine, de borohydrure de sodium ou d'un mélange de ceux-ci est utilisé par rapport au poids de la 1,3-benzoxazine-2,4-dione.

3. Procédé selon l'une quelconque des revendications précédentes, dans lequel l'étape d'hydrolyse en présence de benzotriazole, d'hydrazine, de borohydrure de sodium ou d'un mélange de ceux-ci se produit à une température comprise entre environ 15°C et 40°C.

4. Procédé selon l'une quelconque des revendications précédentes, dans lequel l'étape d'hydrolyse comprend l'ajout d'hydroxyde métallique aqueux et l'agitation à une température élevée jusqu'à environ 95°C.

5. Procédé selon l'une quelconque des revendications précédentes, comprenant en outre le refroidissement à 0 à 5°C suivi de l'ajustement du pH à environ 9 à environ 10, et le lavage de la solution à pH ajusté avec un solvant organique suivi de l'élimination du solvant de lavage organique.

6. Procédé selon la revendication 5, dans lequel l'étape d'isolement comprend l'abaissement du pH de la solution lavée pour obtenir un acide salcaprozique solide humide.

7. Procédé selon l'une quelconque des revendications précédentes, comprenant en outre la filtration et le lavage de l'acide salcaprozique isolé avec un mélange d'acétone et d'eau.

8. Procédé selon l'une quelconque des revendications précédentes, comprenant en outre la mise en contact d'acide salcaprozique, qui est exempt d'impuretés colorées, avec une base organique ou inorganique dans un solvant organique, de l'eau ou un mélange de ceux-ci pour obtenir un sel d'acide salcaprozique.

9. Procédé selon la revendication 8, dans lequel :
a) l'absorbance à 400 nm d'une solution à 1 % du sel d'acide salcaprozique est inférieure à 0,03, par exemple inférieure à 0,02, de préférence d'environ 0,01 ou moins ; et/ou
b) la transmission à 600 nm d'une solution à 1 % du sel d'acide salcaprozique est supérieure à 95 %, par exemple supérieure à 98 %, de préférence d'environ 99 % ou plus.

10. Procédé d'élimination d'une impureté colorée d'un sel d'acide salcaprozique comprenant le traitement d'une solution du sel avec du benzotriazole, de l'hydrazine, du borohydrure de sodium ou un mélange de ceux-ci.

11. Procédé selon la revendication 10, dans lequel l'absorbance à 400 nm d'une solution à 1 % du sel traité est inférieure à 0,03, par exemple inférieure à 0,02, de préférence d'environ 0,01 ou moins.

12. Procédé selon l'une quelconque des revendications précédentes, comprenant :
a. la préparation de l'ester éthylique de l'acide 8-(2,4-dioxo-1,3-benzoxazine-3- yl)octanoïque à partir de la 1,3-benzoxazine-2,4-dione
b. la mise en contact de l'ester éthylique de l'acide 8-(2,4-dioxo-1,3-benzoxazine-3- yl)octanoïque avec environ 1 % à environ 5 % en poids de benzotriazole, d'hydrazine, de borohydrure de sodium ou d'un mélange de ceux-ci par rapport à la quantité de 1,3-benzoxazine-2,4- dione ;
c. l'ajout de l'hydroxyde métallique aqueux et agiter à une température élevée jusqu'à environ 95° C ;
d. le refroidissement à 0 à 5°C suivi d'un ajustement du pH à environ 9 à environ 10 ;
e. le lavage de la solution au pH ajusté avec un solvant organique pour éliminer les sous-produits et les impuretés ; et
f. l'abaissement du pH de la solution lavée pour isoler un acide salcaprozique solide humide.

13. Procédé selon la revendication 12, dans lequel l'étape de mise en contact se produit à une température comprise entre environ 15°C et 40°C.

14. Procédé selon la revendication 12 ou 13, comprenant en outre :
g. la filtration et le lavage de l'acide salcaprozique solide humide avec un mélange d'acétone et d'eau pour obtenir de l'acide salcaprozique purifié ,
dans lequel éventuellement une quantité de n'importe quelle impureté unique dans l'acide salcaprozique purifié est inférieure à 0,1 %.

15. Procédé selon l'une quelconque des revendications 12 à 14, dans lequel :
a) l'acide salcaprozique est exempt d'impuretés colorées ; et/ou
b) une solution à 1 % d'un sel de sodium de l'acide salcaprozique humide présente une absorbance à 400 nm inférieure à 0,03, telle qu'inférieure à 0,02, de préférence d'environ 0,01 ou moins, ou une transmission à 600 nm supérieure à 95 %, telle que supérieure à 98 %, de préférence d'environ 99 % ou plus.
